# FASCICULE DE BREVET EUROPEEN

(11) **EP 3 006 073 B1**
(45) Date de publication et mention de la délivrance du brevet: **18.12.2019**
(21) Numéro de dépôt: 15188696.7
(22) Date de dépôt: 07.10.2015
(51) Int. Cl.: A61M 25/00, A61M 25/06

(54) **TROCART DE RIGIDIFICATION D'UN CATHETER**
TROKAR ZUR VERSTEIFUNG EINES KATHETERS
TROCAR FOR STIFFENING A CATHETER

(30) Priorité: 07.10.2014 FR 1459609
(43) Date de publication de la demande: 13.04.2016
(73) Titulaire: Commissariat à l'Énergie Atomique et aux Énergies Alternatives, 75015 Paris (FR)
(72) Inventeur: CAMPAGNOLO, Raymond, 38100 Grenoble (FR)
(74) Mandataire: Brevalex

(56) Documents cités:
- EP-A1- 1 597 999
- WO-A1-2010/089727
- WO-A2-2008/042285
- US-A1- 2013 096 482

## Description

### DOMAINE TECHNIQUE

La présente invention concerne un trocart apte à coulisser à l'intérieur d'un cathéter destiné à être implanté dans une partie du corps humain, notamment pour l'injection localisée d'un fluide thérapeutique. La présente invention concerne plus particulièrement un trocart apte à coulisser à l'intérieur d'un cathéter destiné à être implanté dans un organe de structure peu compacte du corps humain, tel que le parenchyme cérébral.

### ÉTAT DE LA TECHNIQUE ANTÉRIEURE

Les cathéters présentant une certaine flexibilité, ils ont tendance à dévier de leur trajectoire pendant leur insertion.

La mise en place d'un trocart, généralement en métal, dans un cathéter permet de rendre le cathéter plus rigide pendant son implantation et donc de faciliter son guidage. Une fois le cathéter implanté, le trocart peut être retiré.

De tels trocarts de rigidification sont couramment utilisés pour l'implantation de cathéters dans le réseau vasculaire.

Par ailleurs, le brevet US 8,128,600 B2 décrit un système de guidage d'un cathéter muni d'un trocart pour l'injection localisée d'un agent thérapeutique dans le parenchyme cérébral. Un trocart de faible diamètre, de l'ordre de 0,6 mm, en tungstène est introduit dans le cathéter afin d'augmenter la rigidité du cathéter pendant son implantation. Une fois le cathéter implanté, le trocart peut être extrait pour laisser place au passage de l'agent thérapeutique.

Un inconvénient des systèmes de cathéter muni d'un trocart existants réside dans la formation d'une dépression dans le cathéter lors du retrait du trocart. Ceci est dû au fait que le diamètre du trocart est proche du diamètre interne du cathéter, ce qui rend difficile le pré-remplissage de l'espace interne du cathéter par le fluide à injecter. Il en résulte un risque de formation d'une bulle d'air à l'extrémité du cathéter faisant face à la zone à traiter.

Le document WO 2008/042285 A2 décrit un appareil chirurgical de transfert de fluide.

Le document US 2013/00964832 A1 décrit un trocart pour le positionnement d'un cathéter.

Il se pose donc le problème de prévoir un trocart apte à coulisser à l'intérieur d'un cathéter et permettant un pré-remplissage du volume interne du cathéter par le fluide à injecter avant le retrait du trocart.

### EXPOSÉ DE L'INVENTION

La présente invention vise notamment à résoudre ce problème, qui est résolu par un trocart selon la revendication 1.

La paroi interne d'un cathéter étant généralement de forme cylindrique et de section circulaire, le pré-remplissage du volume interne du cathéter par le fluide à injecter est difficile dans le cas où le trocart est également de forme cylindrique et de section circulaire et de diamètre légèrement inférieur au diamètre interne du cathéter.

La présente invention vise donc à prévoir un trocart de forme particulière permettant un pré-remplissage du volume interne du cathéter par le fluide à injecter avant le retrait du trocart.

La présente invention concerne un trocart comportant une paroi périphérique s'étendant selon un axe longitudinal, dont la section, dans un plan perpendiculaire audit axe longitudinal, est inscrite dans un premier cercle d'un premier diamètre, de sorte que le trocart est apte à coulisser à l'intérieur d'un cathéter comportant une paroi interne présentant des dimensions, par exemple un deuxième diamètre, supérieur(es) ou sensiblement égal(es) audit premier diamètre, le périmètre de ladite section présentant au moins une inflexion, de sorte que, lorsque le trocart coulisse dans le cathéter, au moins un espace entre le cathéter et le trocart à l'emplacement de cette inflexion reste libre.

Par présenter une inflexion, on entend que le rayon de courbure du périmètre de ladite section n'est pas constant, ce qui permet de libérer un espace entre la paroi interne du cathéter et la paroi périphérique du trocart à l'emplacement de cette inflexion.

Un avantage d'un trocart du type de celui décrit ci-dessus, destiné à être inséré dans un cathéter, est lié au fait qu'un espace reste libre dans le volume interne du cathéter lorsque le trocart est mis en place dans le cathéter. Cet espace libre peut contenir le fluide à injecter. Un tel trocart permet donc un pré-remplissage du volume interne du cathéter par le fluide à injecter avant le retrait du trocart. Ceci permet d'éviter la mise en dépression du cathéter lors du retrait du trocart. Il en résulte une élimination du risque de formation d'une bulle d'air à l'extrémité du cathéter, à l'emplacement de la zone à traiter par l'agent thérapeutique.

La présente invention concerne également un trocart comportant une paroi périphérique s'étendant selon un axe longitudinal, dont la section, dans un plan perpendiculaire audit axe longitudinal, est inscrite dans un premier cercle d'un premier diamètre, de sorte que le trocart est apte à coulisser à l'intérieur d'un cathéter comportant une paroi interne présentant un deuxième diamètre supérieur ou sensiblement égal audit premier diamètre, un troisième diamètre d'un second cercle inscrit dans ladite section étant strictement inférieur au deuxième diamètre.

Une partie de la paroi périphérique du trocart peut s'appuyer sur la paroi interne du cathéter.

Ladite inflexion peut être formée d'un méplat. Selon une variante, ladite inflexion peut être formée d'une partie concave.

Le troisième diamètre peut être sensiblement égal à la moitié du deuxième diamètre.

Selon la présente invention, le périmètre de ladite section présente au moins deux inflexions, le trocart comportant en outre un canal central, disposé dans une partie centrale du trocart et s'étendant selon l'axe longitudinal, et au moins deux canaux d'alimentation latéraux s'étendant selon l'axe longitudinal et disposés dans une partie du trocart dont la section est inscrite dans le second cercle, chaque canal d'alimentation latéral débouchant sur le canal central et sur une partie de la paroi périphérique se trouvant à l'emplacement de ladite inflexion correspondante.

Un avantage d'un trocart du type de celui décrit ci-dessus, destiné à être inséré dans un cathéter, réside dans un pré-remplissage plus simple par le fluide à injecter de l'espace libre entre la paroi interne du cathéter et la paroi périphérique du trocart.

Selon un mode de réalisation de la présente invention, le périmètre de ladite section présente trois inflexions régulièrement réparties le long dudit périmètre.

La présente invention concerne en outre un dispositif médical comprenant un trocart du type de celui décrit ci-dessus et un cathéter dans lequel le trocart est apte à coulisser.

### BRÈVE DESCRIPTION DES DESSINS

D'autres caractéristiques et avantages de l'invention ressortiront plus clairement à la lecture de la description suivante et en référence aux dessins annexés, donnés à titre uniquement illustratif et nullement limitatif.
La figure 1 est une vue en coupe représentant de façon schématique un exemple de trocart.
La figure 2 est une vue en coupe représentant de façon schématique un exemple de forme de la section d'un trocart.
La figure 3 est une vue en coupe représentant de façon schématique un autre exemple de forme de la section d'un trocart.
Les figures 4A à 4E sont des vues en coupe représentant de façon schématique d'autres exemples de forme de la section d'un trocart.
La figure 5 est une vue en coupe représentant de façon schématique une variante d'un trocart selon l'invention.

Des parties identiques, similaires ou équivalentes des différentes figures portent les mêmes références numériques de façon à faciliter le passage d'une figure à l'autre.

Les différentes parties représentées sur les figures ne le sont pas nécessairement selon une échelle uniforme, pour rendre les figures plus lisibles.

### EXPOSÉ DÉTAILLÉ DE MODES DE RÉALISATION PARTICULIERS

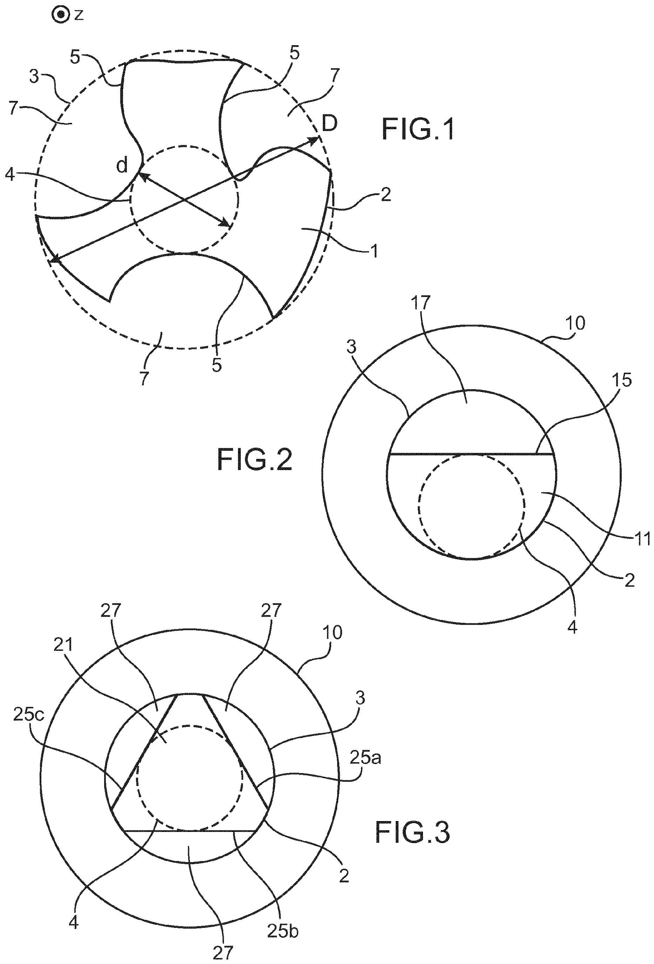

La figure 1 est une vue en coupe représentant de façon schématique un exemple de trocart apte à coulisser à l'intérieur d'un cathéter.

Le trocart 1 comporte une paroi périphérique 2, par exemple de forme cylindrique, s'étendant selon un axe longitudinal z. La figure 1 représente la section du trocart 1 dans un plan perpendiculaire à l'axe longitudinal z.

Selon l'exemple illustré en figure 1, la section du trocart 1 est de forme irrégulière.

Le trocart 1 est apte à coulisser à l'intérieur d'un cathéter comportant une paroi interne présentant un diamètre interne D. La section du trocart 1 est inscrite dans un cercle dont le diamètre est inférieur ou sensiblement égal au diamètre interne D de la paroi interne du cathéter. Le diamètre du cercle dans lequel la section du trocart 1 est inscrite correspond au plus petit diamètre du cercle contenant la section du trocart 1.

Le cathéter est par exemple de forme cylindrique et de section circulaire. La paroi interne du cathéter est représentée par des pointillés 3.

Le cercle représenté par des pointillés 4, de diamètre d, correspond au cercle de plus grand diamètre contenu dans la section du trocart 1, c'est-à-dire le cercle inscrit dans la section du trocart 1.

La forme de la section du trocart 1 sera choisie de sorte que le diamètre d soit inférieur au diamètre interne D du cathéter, et par exemple sensiblement égal à D/2.

Une partie seulement de la paroi périphérique 2 du trocart 1 s'appuie sur la paroi interne 3 du cathéter. Le rayon de courbure du périmètre de la section du trocart 1 n'est pas constant. Le périmètre de la section du trocart 1 présente au moins une inflexion 5.

Dans l'exemple illustré en figure 1, le périmètre de la section du trocart 1 présente trois inflexions 5.

La distance entre la paroi périphérique 2 du trocart 1 et la paroi interne 3 du cathéter augmente aux emplacements des inflexions 5. Ceci permet, aux emplacements des inflexions 5, de libérer un espace 7 entre la paroi interne 3 du cathéter et la paroi périphérique 2 du trocart 1.

De préférence, chaque inflexion 5 de la paroi périphérique 2 du trocart 1 est formée d'une partie concave ou d'un méplat.

Lorsque le trocart 1 coulisse dans le cathéter, l'espace 7 entre le cathéter et le trocart 1 aux emplacements des inflexions 5 reste libre, tandis qu'une partie de la paroi périphérique 2 du trocart 1 s'appuie sur ladite paroi interne 3 du cathéter.

Un avantage d'un trocart du type de celui décrit en relation avec la figure 1, destiné à être inséré dans un cathéter, est lié au fait qu'un espace reste libre dans le volume interne du cathéter lorsque le trocart est mis en place dans le cathéter. Cet espace libre peut contenir le fluide à injecter. Un tel trocart permet donc un pré-remplissage du volume interne du cathéter par le fluide à injecter avant le retrait du trocart. Ceci permet d'éviter la mise en dépression du cathéter lors du retrait du trocart. Il en résulte une réduction du risque de formation d'une bulle d'air à l'extrémité du cathéter, à l'emplacement de la zone à traiter par l'agent thérapeutique.

Une solution pour réaliser un trocart tel que celui décrit en relation avec la figure 1 est de partir d'un trocart présentant une section circulaire de diamètre sensiblement égal au diamètre D du cathéter, et de réaliser un ou plusieurs méplats.

La figure 2 est une vue en coupe représentant de façon schématique un exemple de forme de la section d'un trocart 11 apte à coulisser à l'intérieur d'un cathéter 10. Selon cet exemple, le trocart 11 comprend un méplat 15. Ceci permet de libérer un espace 17 entre la paroi interne 3 du cathéter 10 et la paroi périphérique 2 du trocart 11.

La figure 3 est une vue en coupe représentant de façon schématique un autre exemple de forme de la section d'un trocart 21 apte à coulisser à l'intérieur d'un cathéter 10. Selon cet exemple, le trocart 21 comprend trois méplats 25a, 25b, 25c. Ceci permet de libérer un espace 27 entre la paroi interne 3 du cathéter 10 et la paroi périphérique 2 du trocart 21, à l'emplacement de chacun des méplats 25a, 25b, 25c.

D'autres formes de trocart, comportant par exemple un plus grand nombre de méplats, sont possibles.

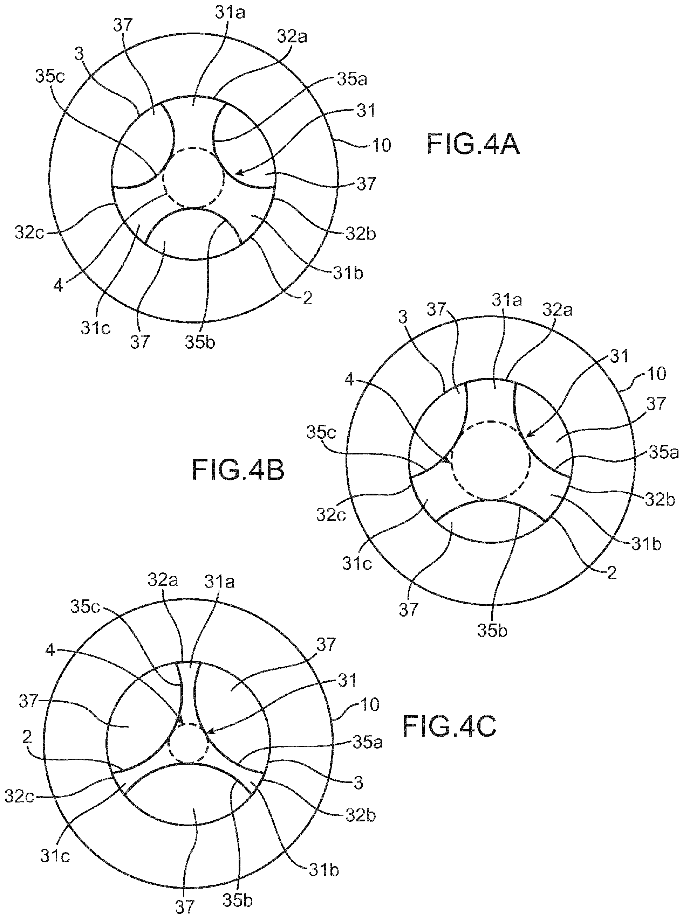

Les figures 4A à 4E sont des vues en coupe représentant de façon schématique d'autres exemples de forme de la section d'un trocart apte à coulisser à l'intérieur d'un cathéter 10.

La figure 4A représente un exemple de forme de la section d'un trocart 31 comportant trois branches 31a, 31b, 31c. Dans chaque branche 31a, 31b, 31c, une partie 32a, 32b, 32c de la paroi périphérique 2 du trocart 31 s'appuie sur la paroi interne 3 du cathéter 10. La paroi périphérique 2 du trocart 31 comprend trois parties concaves 35a, 35b, 35c régulièrement réparties le long de la paroi périphérique 2. Les parties concaves 35a, 35b, 35c relient respectivement les parties 32a et 32b, 32b et 32c, 32c et 32a de la paroi périphérique 2. Ceci permet de libérer un espace 37 entre la paroi interne 3 du cathéter 10 et la paroi périphérique 2 du trocart 31, à l'emplacement de chacune des parties concaves 35a, 35b, 35c.

Les figures 4B et 4C représentent deux autres exemples de forme de la section d'un trocart 31 comprenant trois branches 31a, 31b, 31c et trois parties concaves 35a, 35b, 35c régulièrement réparties le long de la paroi périphérique 2. Selon l'exemple illustré en figure 4B, le diamètre d du cercle 4 de plus grand diamètre contenu dans la section du trocart 31 est supérieur au diamètre d dans le cas de l'exemple illustré en figure 4A. Selon l'exemple illustré en figure 4C, le diamètre d du cercle 4 de plus grand diamètre contenu dans la section du trocart 31 est inférieur au diamètre d dans le cas de l'exemple illustré en figure 4A.

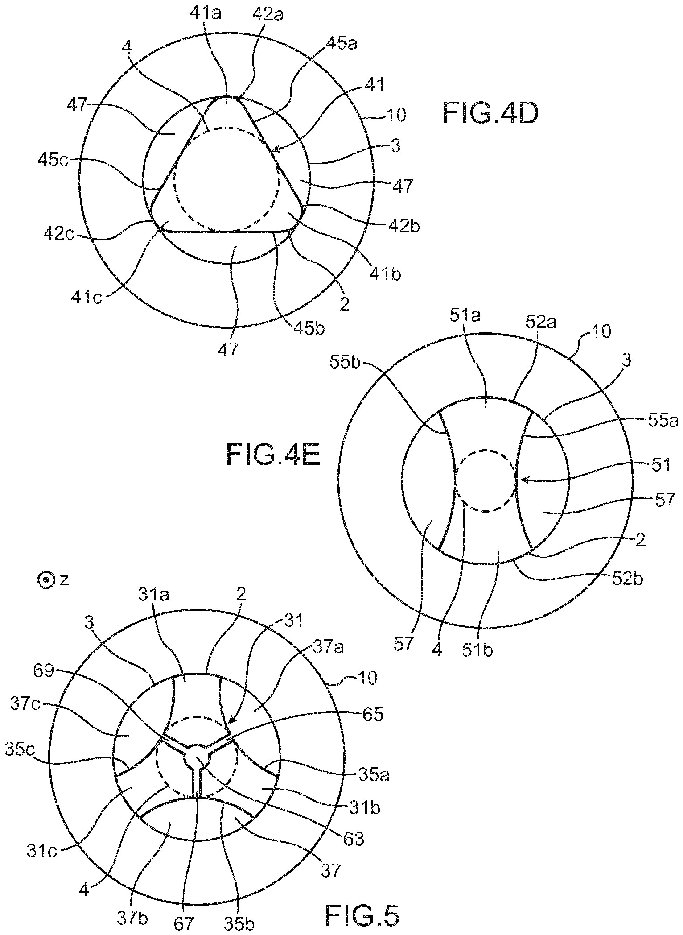

La figure 4D représente un autre exemple de forme de la section d'un trocart 41 comprenant trois branches 41a, 41b, 41c. Dans chaque branche 41a, 41b, 41c, une partie 42a, 42b, 42c de la paroi périphérique 2 du trocart 41 s'appuie sur la paroi interne 3 du cathéter 10. La paroi périphérique 2 du trocart 41 comprend trois méplats 45a, 45b, 45c régulièrement répartis le long de la paroi périphérique 2. Les méplats 45a, 45b, 45c relient respectivement les parties 42a et 42b, 42b et 42c, 42c et 42a de la paroi périphérique 2. Ceci permet de libérer un espace 47 entre la paroi interne 3 du cathéter 10 et la paroi périphérique 2 du trocart 41, à l'emplacement de chacun des méplats 45a, 45b, 45c.

D'autres formes de trocart, comportant par exemple un nombre différent de branches, sont possibles.

La figure 4E représente un exemple de forme de la section d'un trocart 51 comportant deux branches 51a et 51b. Dans chaque branche 51a, 51b, une partie 52a, 52b de la paroi périphérique 2 du trocart 51 s'appuie sur la paroi interne 3 du cathéter 10. La paroi périphérique 2 du trocart 51 comporte deux parties concaves 55a et 55b reliant les parties 52a et 52b. Ceci permet de libérer un espace 57 entre la paroi interne 3 du cathéter 10 et la paroi périphérique 2 du trocart 51, à l'emplacement de chaque partie concave 55a, 55b.

D'une manière générale, il suffit de respecter la règle édictée pour la section du trocart, c'est-à-dire que le cercle de plus grand diamètre contenu dans la section du trocart présente un diamètre d inférieur au diamètre interne D du cathéter, le plus petit diamètre du cercle contenant la section du trocart étant inférieur ou sensiblement égal à D.

On choisira par exemple un diamètre d de l'ordre de D/2 de façon que la rigidité du trocart soit suffisante pour rigidifier le cathéter lors de son introduction.

En outre, la section du trocart peut ne pas être uniforme. Par exemple, la section du trocart peut présenter la forme d'une hélice de section non uniforme.

La figure 5 est une vue en coupe représentant de façon schématique une variante d'un trocart 31 selon l'invention qui est apte à coulisser à l'intérieur d'un cathéter 10. Cette variante est décrite dans le cas d'un trocart 31 comprenant trois branches 31a, 31b, 31c et trois parties concaves 35a, 35b, 35c régulièrement réparties le long de la paroi périphérique 2. Bien entendu, cette variante peut s'appliquer à un trocart comprenant un nombre quelconque de branches supérieur ou égal à deux.

Selon cette variante, un canal central 63, s'étendant selon l'axe longitudinal z, est disposé dans la partie centrale du trocart 31. Des canaux d'alimentation latéraux 65, 67, 69, s'étendant selon l'axe longitudinal z, sont disposés dans la partie du trocart 31 contenue dans le cercle 4 de diamètre d. Chaque canal d'alimentation latéral 65, 67, 69 débouche sur le canal central 63 et sur une partie de la paroi périphérique 2 se trouvant respectivement à l'emplacement de la partie concave 35a, 35b, 35c. Le canal central 63 et les canaux d'alimentation latéraux 65, 67, 69 sont destinés à être traversés par le fluide à injecter.

L'espace libre 37 entre le trocart 31 et le cathéter 10 comprend trois portions 37a, 37b, 37c séparées par les branches 31a, 31b, 31c du trocart 31. Chaque portion 37a, 37b, 37c est disposée entre la paroi interne 3 du cathéter 10 et la partie concave 35a, 35b, 35c correspondante de la paroi périphérique 2 du trocart 31. Grâce au canal central 63 et aux canaux d'alimentation latéraux 65, 67, 69, le fluide à injecter peut être introduit par le canal central 63 et passer dans les canaux d'alimentation latéraux 65, 67, 69, ce qui permet de remplir les trois portions 37a, 37b, 37c de l'espace libre 37 entre le trocart 31 et le cathéter 10.

Un avantage d'une telle variante réside dans un pré-remplissage plus simple par le fluide à injecter de l'espace libre 37 entre la paroi interne 3 du cathéter 10 et la paroi périphérique 2 du trocart 31.

Bien que la variante de la figure 5 ait été décrite dans le cas où les inflexions de la paroi périphérique du trocart sont formées de parties concaves, cette variante s'applique bien entendu au cas où ces inflexions sont formées de méplats.

Les trocarts et les dispositifs médicaux associant les trocarts aux cathéters précédemment décrits sont avantageusement destinés à être implantés dans un parenchyme cérébral pathologique (neuro-dégénératif ou tumoral) pour la délivrance localisée d'un principe thérapeutique, par exemple d'un liquide thérapeutique anti-tumoral.

## Revendications

1. Trocart (1, 21, 31, 41, 51) comportant une paroi périphérique (2) s'étendant selon un axe longitudinal (z), dont la section, dans un plan perpendiculaire audit axe longitudinal, est inscrite dans un premier cercle d'un premier diamètre, de sorte que le trocart est apte à coulisser à l'intérieur d'un cathéter (10) comportant une paroi interne (3), de section circulaire, présentant un deuxième diamètre (D) supérieure ou sensiblement égale audit premier diamètre,
le périmètre de ladite section présentant au moins deux inflexions (5 ; 25a, 25b, 25c ; 35a, 35b, 35c ; 45a, 45b, 45c ; 55a, 55b), de sorte que, lorsque le trocart coulisse dans le cathéter (10), au moins deux espaces (7, 27, 37, 47, 57) entre le cathéter et le trocart aux emplacements des au moins deux inflexions restent libres,
dans lequel un troisième diamètre (d) d'un second cercle (4) inscrit dans ladite section est strictement inférieur deuxième diamètre (D) de la paroi interne (3) du cathéter (10),
le trocart comportant en outre :
un canal central (63) disposé dans une partie centrale du trocart (31) et s'étendant selon l'axe longitudinal (z) ; et
au moins deux canaux d'alimentation latéraux (65, 67, 69) s'étendant selon l'axe longitudinal (z) et disposés dans une partie du trocart dont la section est inscrite dans le second cercle (4),
et dans lequel chaque canal d'alimentation latéral débouche sur le canal central et sur une partie de la paroi périphérique (2) se trouvant à l'emplacement de ladite inflexion correspondante (5 ; 25a, 25b, 25c ; 35a, 35b, 35c ; 45a, 45b, 45c ; 55a, 55b).

2. Trocart selon la revendication 1, dans lequel chacune des deux inflexions est formée d'un méplat (25a, 25b, 25c ; 45a, 45b, 45c) ou d'une partie concave (35a, 35b, 35c; 55a, 55b).

3. Trocart selon la revendication 1 ou 2, dans lequel le périmètre de ladite section présente trois inflexions (5 ; 25a, 25b, 25c ; 45a, 45b, 45c ; 35a, 35b, 35c) régulièrement réparties le long dudit périmètre.

4. Dispositif médical comprenant un trocart (1, 21, 31, 41, 51) selon l'une des revendications 1 à 3 et un cathéter (10) dans lequel le trocart est apte à coulisser.

5. Dispositif médical selon la revendication 4, dans lequel le troisième diamètre (d) est sensiblement égal à la moitié du deuxième diamètre (D) de la paroi interne (3) du cathéter (10).

## Patentansprüche

1. Trokar (1, 21, 31, 41, 51), umfassend eine Umfangswand (2), welche sich entlang einer longitudinalen Achse (z) erstreckt, deren Querschnitt in einer Ebene senkrecht zu der longitudinalen Achse in einen ersten Kreis mit einem ersten Durchmesser eingeschrieben ist, so dass der Trokar in der Lage ist, im Inneren eines Katheters (10) zu gleiten, welcher eine innere Wand (3) von kreisförmigem Querschnitt aufweist, welcher einen zweiten Durchmesser (D) aufweist, welcher größer oder im Wesentlichen gleich zu dem ersten Durchmesser ist,
wobei der Durchmesser des Querschnitts wenigstens zwei Biegungen (5; 25a, 25b, 25c; 35a, 35b, 35c; 45a, 45b, 45c; 55a, 55b) derart aufweist, dass wenn der Trokar in dem Katheter (10) gleitet, wenigstens zwei Räume (7, 27, 37, 47, 57) zwischen dem Katheter und dem Trokar an den Stellen der wenigstens zwei Biegungen frei bleiben,
wobei ein dritter Durchmesser (d) eines zweiten Kreises (4), welcher in dem Querschnitt eingeschrieben ist, streng kleiner als der zweite Durchmesser (D) der inneren Wand (3) des Katheters (10) ist,
wobei der Trokar ferner umfasst:
einen zentralen Kanal (63), welcher in einem zentralen Abschnitt des Trokars (31) angeordnet ist und sich entlang der longitudinalen Achse (z) erstreckt; und wenigstens zwei laterale Versorgungskanäle (65, 67, 69), welche sich entlang der longitudinalen Achse (z) erstrecken und in einem Abschnitt des Trokar angeordnet sind, dessen Querschnitt in dem zweiten Kreis (4) eingeschrieben ist,
und wobei jeder laterale Versorgungskanal in den zentralen Kanal und an einem Abschnitt der Umfangswand (2) mündet, welcher sich an der Stelle der entsprechenden Biegung (5; 25a, 25b, 25c; 35a, 35b, 35c; 45a, 45b, 45c; 55a, 55b) befindet.

2. Trokar nach Anspruch 1, wobei jede der beiden Biegungen durch eine Abflachung (25a, 25b, 25c; 45a, 45b, 45c) oder einen konkaven Abschnitt (35a, 35b, 35c; 55a, 55b) gebildet ist.

3. Trokar nach Anspruch 1 oder 2, wobei der Umfang des Querschnitts drei Biegungen (5; 25a, 25b, 25c; 45a, 45b, 45c; 35a, 35b, 35c) aufweist, welche entlang des Umfangs gleichmäßig verteilt sind.

4. Medizinische Vorrichtung, umfassend einen Trokar (1, 21, 31, 41, 51) nach einem der Ansprüche 1 bis 3 und einen Katheter (10), in welchem der Trokar in der Lage ist, zu gleiten.

5. Medizinische Vorrichtung nach Anspruch 4, wobei der dritte Durchmesser (d) im Wesentlichen gleich der Hälfte des zweiten Durchmessers (D) der inneren Wand (3) des Katheters (10) ist.

## Claims

1. A trocar (1, 21, 31, 41, 51) including a peripheral wall (2) extending along a longitudinal axis (z), the section of which, in a plane perpendicular to said longitudinal axis, is inscribed in a first circle with a first diameter, so that the trocar is able to slide inside a catheter (10) including an inner wall (3), with a circular section, having a second diameter (D) greater than or substantially equal to said first diameter,
the perimeter of said section having at least two inflections (5; 25a, 25b, 25c; 35a, 35b, 35c; 45a, 45b, 45c; 55a, 55b), so that, when the trocar slides in the catheter (10), at least two spaces (7, 27, 37, 47, 57) between the catheter and the trocar at the locations of at least two inflections remain free,
wherein a third diameter (d) of a second circle (4) inscribed in said section is strictly lower than the second diameter (D) of the inner wall (3) of the catheter (10),
the trocar further including:
a centre channel (63) disposed in a centre part of the trocar (31) and extending along the longitudinal axis (z); and
at least two supplying side channels (65, 67, 69) extending along the longitudinal axis (z) and disposed in a part of the trocar the section of which is inscribed in the second circle (4),
and wherein each supplying side channel comes out on the centre channel and on a part of the peripheral wall (2) being at the location of said corresponding inflection (5; 25a, 25b, 25c; 35a, 35b, 35c; 45a, 45b, 45c; 55a, 55b).

2. The trocar according to claim 1, wherein each of both inflections is formed of a flat surface (25a, 25b, 25c; 45a, 45b, 45c) or of a concave part (35a, 35b, 35c; 55a, 55b).

3. The trocar according to claim 1 or 2, wherein the perimeter of said section has three inflections (5; 25a, 25b, 25c; 45a, 45b, 45c; 35a, 35b, 35c) evenly distributed along said perimeter.

4. A medical device comprising a trocar (1, 21, 31, 41, 51) according to one of claims 1 to 3 and a catheter (10) in which the trocar is able to slide.

5. The medical device according to claim 4, wherein the third diameter (d) is substantially equal to half the second diameter (D) of the inner wall (3) of the catheter(10).
